# EUROPEAN PATENT SPECIFICATION

(11) **EP 3 858 312 B1**
(45) Date of publication and mention of the grant of the patent: **15.02.2023**
(21) Application number: 19867934.2
(22) Date of filing: 27.09.2019
(51) Int. Cl.: A61F 13/47, A61F 5/44, A61F 13/15, A61F 13/471, A61F 13/532, A61F 13/56

(54) **ABSORBENT ARTICLE FOR MEN**
SAUGFÄHIGER ARTIKEL FÜR MÄNNER
PROTECTION ABSORBANTE POUR HOMME

(30) Priority: 27.09.2018 JP 2018181562
(43) Date of publication of application: 04.08.2021
(73) Proprietor: DAIO PAPER CORPORATION, Shikokuchuo-shi Ehime 799-0492 (JP)
(72) Inventor: TAKAHIRA, Akira, Sakura-shi, Tochigi 329-1411 (JP)
(74) Representative: Bandpay & Greuter
(86) International application number: PCT/JP2019/038238
(87) International publication number: WO 2020/067461

(56) References cited:
- EP-B1- 1 549 272
- EP-B1- 3 047 827
- WO-A1-2011/162657
- WO-A1-2018/159498
- JP-A- S61 217 160
- JP-A- 2005 532 110

## Description

### Technical Field

The present invention relates to an absorbent article for men, which is particularly suitable for light incontinence of men, and specifically relates to an absorbent article for men in which a plurality of pairs of three-dimensional deformation guiding grooves having a substantially line-symmetric shape with respect to a vertical center line is provided on both sides of a main body member in a vertical direction.

### Background Art

Conventionally, there has been known an absorbent article provided with a deformation guiding groove that guides deformation of a main body member to fit to a body of a wearer and prevent leakage.

For example, Patent Document 1 below discloses an absorbent pad for men in which a pad body has a bending guide groove that is concave from a skin-facing surface to a non-skin-facing surface side, the bending guide groove has a first groove that intersects a vertical axis and extends obliquely and a second groove that intersects the vertical axis and extends in a direction intersecting the first groove, and an intersection of the first groove and the second groove is located on the vertical axis.

In addition, Patent Document 2 below discloses an absorbent pad for men in which a plurality of deformation guide portions is arranged mutually at intervals in a longitudinal direction and a horizontal direction only in a middle region of a pad body.

### Citation List

### Patent Document

Patent Document 1: JP-A-2015-58325
Patent Document 2: JP-A-2017-6303

### Summary of the Invention

### Technical Problem

However, in the absorbent articles for men described in Patent Documents 1 and 2, the bending guide groove and the deformation guide portions, which are compacted and hardened by embossing, are formed in a portion coming into contact with the penis during wearing, and thus there is concern that a wearer may feel awkward or uncomfortable.

In addition, in the absorbent article for men described in Patent Document 1, during wearing, the first groove and the second groove radially located around an intersection M are bent, and a portion located therebetween becomes a peripheral wall and deforms to have a cup shape having the intersection M as an apex. However, in the cup shape having the intersection M as the apex, fitting to a body of the wearer is difficult, and wearing feeling may not be satisfactory. Meanwhile, in the absorbent article for men described in Patent Document 2, it is considered that by bending a peripheral part between a deformation guide portion and a deformation guide portion, deformation into a curved shape along the body of the wearer easily occurs. However, since the deformation guide portion is formed in a dot shape, deformation into a convex shape using this dot-shaped deformation guide portion as an apex easily occurs, and there is a risk of a similar problem to that of Patent Document 1.

Therefore, a main object of the invention is to provide an absorbent article for men, which does not give the wearer a feeling of awkwardness or discomfort and is easily fit to the body.

### Solution to Problem

To solve the above-mentioned problem, as the invention according to claim 1, provided is an absorbent article for men having a vertical direction and a width direction orthogonal to the vertical direction, the absorbent article including
a main body member including an absorber,
characterized in that a plurality of pairs of three-dimensional deformation guiding grooves having a substantially line-symmetric shape with respect to a vertical center line is provided at intervals in the vertical direction in regions on both sides in the width direction divided in two parts by the vertical center line,
each of the three-dimensional deformation guiding grooves is formed in a shape gradually inclined outward in the width direction toward an upper side, and an inclination angle of a three-dimensional deformation guiding groove arranged on a lower side is larger than an inclination angle of a three-dimensional deformation guiding groove arranged on the upper side, the inclination angle being an angle formed by a direction in which the three-dimensional deformation guiding groove extends and a width direction line, and
one three-dimensional deformation guiding groove and the other three-dimensional deformation guiding groove forming a pair are arranged apart to a right and a left at a center of the absorbent article for men in the width direction.

In the invention according to claim 1, a plurality of pairs of three-dimensional deformation guiding grooves having a substantially line-symmetric shape with respect to a vertical center line is provided at intervals in the vertical direction on both sides of the main body member in the vertical direction. Since each of the three-dimensional deformation guiding grooves is formed in a shape gradually inclined outward in the width direction toward an upper side in the vertical direction, three-dimensional deformation guiding grooves forming a right-left pair are formed in a substantially V-shape. In this instance, an inclination angle (an angle formed by a direction in which the three-dimensional deformation guiding groove extends and a width direction line) of a three-dimensional deformation guiding groove arranged on a lower side is larger than an inclination angle of a three-dimensional deformation guiding groove arranged on the upper side. In addition, one three-dimensional deformation guiding groove and the other three-dimensional deformation guiding groove forming a pair are arranged apart to a right and a left at a center of the absorbent article for men in the width direction. For this reason, since the three-dimensional deformation guiding groove is not formed in the widthwise central portion touched by the penis in a state where absorbent article for men is worn, a wearer does not feel awkward or uncomfortable. In addition, when the penis is pressed against the skin contact surface side of the widthwise central portion, the widthwise central portion is deformed so as to bulge toward the non-skin side from the three-dimensional deformation guiding groove as a starting point. However, in this instance, since the inclination angle of the three-dimensional deformation guiding groove on the lower side in the vertical direction is large, and the inclination angle decreases stepwise toward the upper side, both sides of the three-dimensional space of the widthwise central portion accommodating the penis are likely to bend in a direction close to the vertical direction on the lower side, and likely to bend in a direction close to the width direction toward the upper side. For this reason, a cross-sectional shape of the three-dimensional space of the widthwise central portion has a small width and a high bulging height to the non-skin side on the lower side, and has a large width and a low bulging height to the non-skin side on the upper side. Therefore, the penis easily fits properly to the three-dimensional space having such a cross-sectional shape, and the wearer does not feel awkward or uncomfortable.

As the invention according to claim 2, provided is the absorbent article for men according to claim 1, in which a separating distance of a three-dimensional deformation guiding groove arranged on the lower side is smaller than a separating distance of a three-dimensional deformation guiding groove arranged on the upper side, the separating distance being a distance in the width direction between a widthwise inner end of the three-dimensional deformation guiding groove and the vertical center line.

In the invention according to claim 2, a widthwise inner end of the three-dimensional deformation guiding groove serving as a starting point of three-dimensional deformation in the widthwise central portion of the main body member is disposed to be located on the outer side in the width direction toward the upper side. Thus, when the absorbent article for men is worn, a three-dimensional space having a shape easily fit to the body is likely to be formed in the widthwise central portion, the three-dimensional deformation guiding groove is less likely to touch the penis more reliably during wearing, and the wearer does not feel awkward or uncomfortable.

As the invention according to claim 3, provided is the absorbent article for men according to claim 1 or 2, in which three-dimensional deformation guiding grooves adjacent to each other in the vertical direction are arranged at positions not overlapping in the width direction.

In the invention according to claim 3, by arranging three-dimensional deformation guiding grooves adjacent to each other in the vertical direction at positions not overlapping in the width direction, formation of a three-dimensional space having a stepwise cross-section shape in the widthwise central portion is not hindered when the absorbent article for men is worn.

As the invention according to claim 4, provided is the absorbent article for men according to any one of claims 1 to 3, in which a leakage prevention blocking groove extending substantially in the width direction is arranged in the main body member in one or both of regions on an upper side or a lower side of a vertical section in which the plurality of pairs of three-dimensional deformation guiding grooves is formed.

In the invention according to claim 4, by forming a leakage prevention blocking groove extending substantially in the width direction in the main body member in one or both of regions on an upper side or a lower side of a vertical section in which the plurality of pairs of three-dimensional deformation guiding grooves is formed, the body fluid is prevented from diffusing to the outer side of the leakage prevention blocking groove, and leakage from the end edge of the main body member in the vertical direction is prevented.

As the invention according to claim 5, provided is the absorbent article for men according to any one of claims 1 to 4, in which a shift prevention layer for fixing to underwear is provided on a non-skin contact surface of the absorbent article for men, and
the shift prevention layer is provided along the vertical direction in a part overlapping a widthwise central portion, from which the three-dimensional deformation guiding grooves forming the pair are separated to the right and left, in a thickness direction.

In the invention according to claim 5, since the shift prevention layer is provided along the vertical direction in a part overlapping a widthwise central portion, from which the three-dimensional deformation guiding grooves forming the pair are separated to the right and left, in a thickness direction, deformation of the widthwise central portion from the three-dimensional deformation guiding groove as a starting point is less likely to be hindered when the absorbent article for men is worn.

As the invention according to claim 6, provided is the absorbent article for men according to any one of claims 1 to 5, in which in the absorber, a low basis weight portion is formed along the vertical direction in the widthwise central portion, from which the three-dimensional deformation guiding grooves forming the pair are separated to the right and left.

In the invention according to claim 6, since a low basis weight portion having a relatively low basis weight of the absorber is formed along the vertical direction in the widthwise central portion, from which the three-dimensional deformation guiding grooves forming the pair are separated to the right and left, this region is more likely to be three-dimensionally deformed, and the penis is more likely to fit to this three-dimensionally deformed portion when the absorbent article for men is worn.

As the invention according to claim 7, provided is the absorbent article for men according to any one of claims 1 to 6, in which the absorbent article for men is folded in the vertical direction at one or a plurality of fold lines spaced in the vertical direction along the width direction in an item packaging state, and
the fold line is provided at a position not intersecting the three-dimensional deformation guiding groove.

In the invention according to claim 7, to facilitate three-dimensional deformation by the three-dimensional deformation guiding groove when the absorbent article for men is worn, the fold line in the item packaging state is provided at a position not intersecting the three-dimensional deformation guiding groove.

### Advantageous Effects of the Invention

As described above in detail, according to the invention, it becomes easy to fit to a body without giving a wearer a feeling of awkwardness or discomfort.

### Brief Description of the Drawings

Fig. 1 is a partially broken development view of an absorbent article for men 1 according to the invention.
Fig. 2 is a diagram taken along line II-II of Fig. 1.
Fig. 3 is a perspective view schematically illustrating a shape during wearing.
Fig. 4(A) is a diagram taken along line IVA-IVA of Fig. 3, and Fig. 4(B) is a diagram taken along line IVB-IVB of Fig. 3.

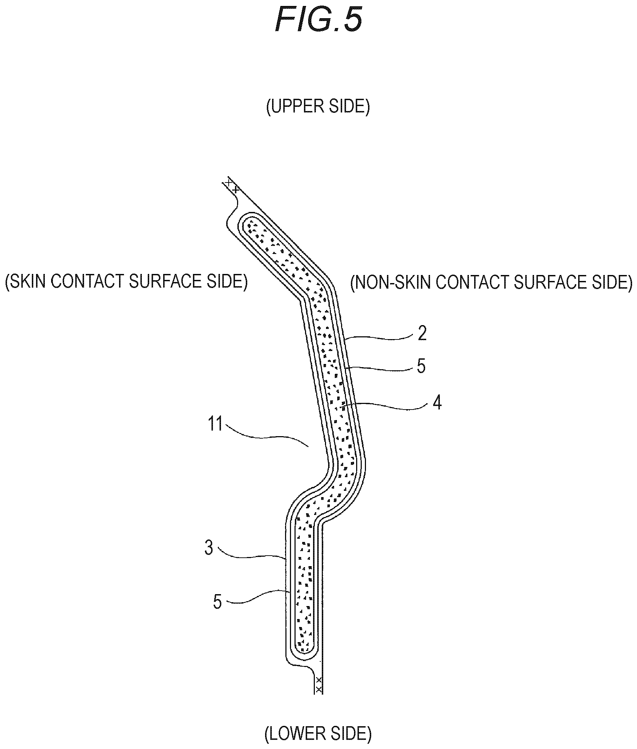

Fig. 5 is a diagram taken along line V-V of Fig. 3.
Fig. 6 is a plan view of an absorbent article for men 1 according to a modification.
Fig. 7 is a plan view of an absorbent article for men 1 according to a modification.
Fig. 8 is a diagram taken along line VIII-VIII of Fig. 6(B) in a worn state.
Fig. 9 is a plan view of a non-skin contact surface side of an absorbent article for men 1 according to a modification.
Fig. 10 is a plan view of an absorbent article for men 1 according to a modification.
Fig. 11 is a diagram taken along line XI-XI of Fig. 10.
Fig. 12 is a plan view of an absorbent article for men 1 according to a modification.

### Mode for Carrying Out the Invention

Hereinafter, an embodiment of the invention will be described in detail with reference to the drawings.

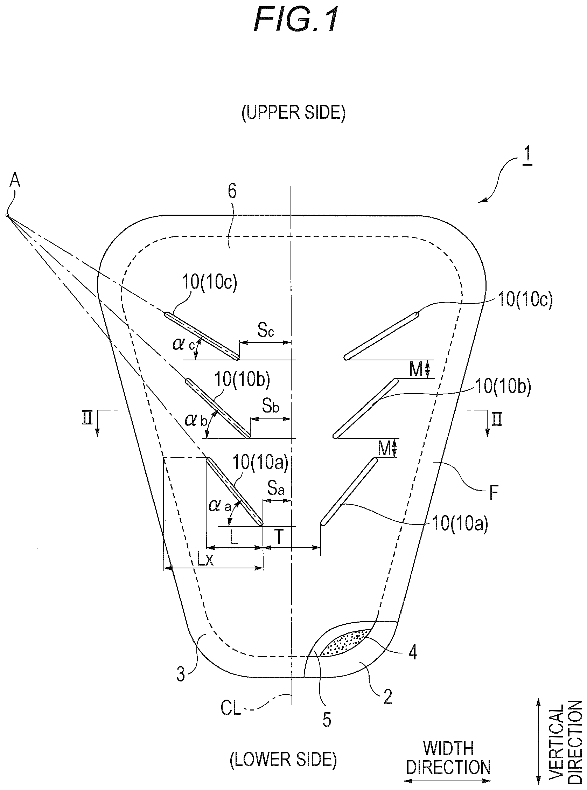

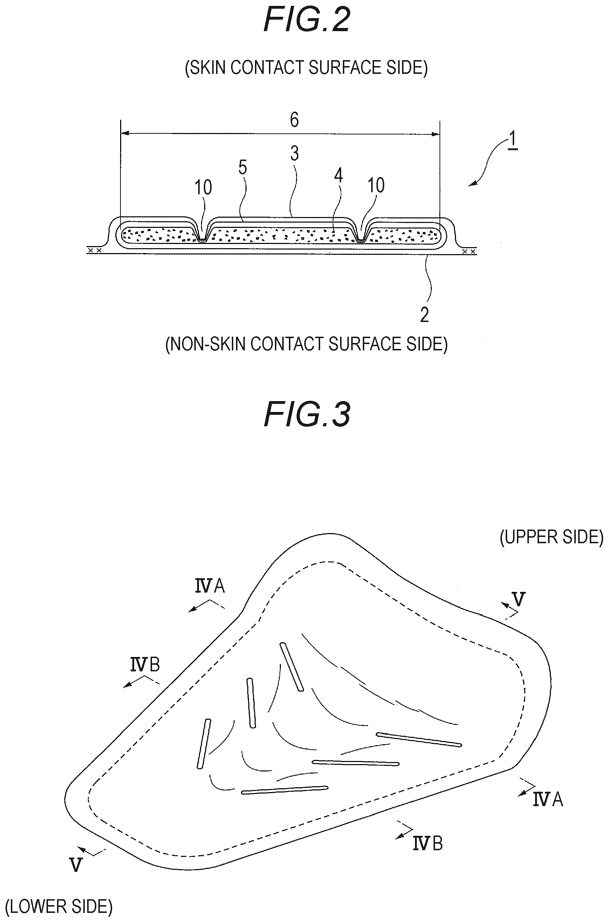

As illustrated in Figs. 1 and 2, an absorbent article for men 1 according to the invention includes a liquid-impermeable back sheet 2 made of a polyethylene sheet, a polypropylene sheet etc., a liquid-permeable face sheet 3 that allows rapid permeation of a body fluid such as urine, an absorber 4 made of fluff pulp or synthetic pulp interposed between these sheets 2 and 3, and an encapsulation sheet 5 made of crepe paper or a nonwoven fabric covering at least a skin side surface and a non-skin side surface of the absorber 4 in order to maintain a shape of the absorber 4 and improve diffusibility. In addition, around a main body member 6 in which the absorber 4 is interposed, the liquid-impermeable back sheet 2 and the liquid-permeable face sheet 3 are joined at outer edge portions by an adhesive such as hot melt or joining means such as heat sealing or ultrasonic sealing, and a flap portion F where the absorber 4 does not exist is formed on an outer periphery.

As illustrated in Fig. 1, the absorbent article for men 1 has a vertical direction corresponding to a vertical direction of a wearer during wearing and a width direction orthogonal thereto. The absorbent article for men 1 has a vertically long shape in which a maximum length in the vertical direction is longer than that in the width direction in the plan view illustrated in Fig. 1. A planar shape of the absorbent article for men 1 is not particularly limited as long as the planar shape is a shape that fits to a crotch portion. However, to improve fitting to the crotch portion, it is preferable to adopt a substantially trapezoidal shape having a wide width on the upper side and a narrow width on the lower side. Additionally, it is possible to adopt an inverted triangular shape, a circular shape, an elliptical shape, a polygonal shape, etc. An outer width of the absorbent article for men 1 on the upper side is preferably set to 100 to 150 mm, and the outer width on the lower side is preferably set to 60 to 80 mm. The absorbent article for men 1 has a substantially line-symmetrical shape with respect to a vertical center line CL that divides the absorbent article for men 1 into two parts in the width direction and extends in the vertical direction.

Hereinafter, a structure of the absorbent article for men 1 will be further described in more detail.

The liquid-impermeable back sheet 2 is made of a sheet material having at least a water-blocking property such as an olefin resin sheet such as polyethylene or polypropylene. However, in addition to this material, it is possible to use a laminated nonwoven fabric obtained by laminating a nonwoven fabric on a polyethylene sheet, etc. or a nonwoven fabric sheet (in this case, a waterproof film and the nonwoven fabric are included in the liquid-impermeable back sheet), etc. after ensuring a substantially liquid impermeability by interposing the waterproof film. In recent years, a material having moisture permeability tends to be used from a viewpoint of preventing stuffiness. This water-blocking/moisture-permeable sheet material is a microporous sheet obtained by melt-kneading an inorganic filler in an olefin resin such as polyethylene or polypropylene to form a sheet, and then stretching the sheet in a uniaxial or biaxial direction.

Next, as the liquid-permeable face sheet 3, a perforated or non-perforated nonwoven fabric, a porous plastic sheet, etc. is preferably used. As a material fiber included in the nonwoven fabric, in addition to synthetic fibers such as olefins such as polyethylene and polypropylene, polyesters, and polyamides, it is possible to use recycled fibers such as rayon and cupra, and natural fibers such as cotton, and it is possible to use a nonwoven fabric obtained by an appropriate processing method such as a spunlace method, a spunbond method, a thermal bond method, a meltblown method, or a needle punch method. Among these processing methods, the spunlace method is excellent in flexibility, the spunbond method is excellent in drapability, and the thermal bond method and an air-through method are excellent in bulkiness and high compression recovery. The fibers of the nonwoven fabric may be either long fibers or short fibers, and preferably short fibers are used in order to give the texture of a towel cloth. Further, in order to facilitate an embossing treatment, it is preferable to use an olefin fiber having a relatively low melting point such as polyethylene or polypropylene. Further, it is possible to preferably use a core-sheath fiber having a high melting point fiber as a core and a low melting point fiber as a sheath, a side-by-side type fiber, or a composite fiber such as a split type fiber.

As the absorber 4 interposed between the liquid-impermeable back sheet 2 and the liquid-permeable face sheet 3, for example, an absorber in which super absorbent resin is mixed in pulp, or an absorber in which chemical fibers are mixed and super absorbent resin is mixed in pulp is used. As illustrated in the figure, it is desirable that the absorber 4 is covered with the encapsulation sheet 5 in order to maintain the shape and rapidly diffuse the body fluid, and prevent the body fluid once absorbed from returning. Examples of the pulp include chemical pulp obtained from wood and pulp such as dissolving pulp containing cellulose fibers or artificial cellulose fibers such as rayon and acetate. Softwood pulp having a long fiber length is more preferably used than hardwood pulp in terms of function and cost. In addition, as the absorber 4, it is possible to use an air-laid absorber which can reduce the bulk or a polymer sheet in which a super absorbent resin is arranged between two layers of nonwoven fabrics.

Further, the absorber 4 may be mixed with a synthetic fiber. As the synthetic fiber, for example, it is possible to use a polyolefin-based material such as polyethylene or polypropylene, a polyester-based material such as polyethylene terephthalate or polybutylene terephthalate, a polyamide-based material such as nylon, or a copolymer thereof, and it is possible to use a mixture of two of these types. In addition, it is possible to use a core-sheath fiber having a high melting point fiber as a core and a low melting point fiber as a sheath, a side-by-side type fiber, or a composite fiber such as a split type fiber. In the case where the synthetic fiber is a hydrophobic fiber, it is desirable to use the synthetic fiber which is surface-treated using a hydrophilizing agent to have an affinity for the body fluid.

Examples of the super absorbent resin include crosslinked polyacrylic acid salt, self-crosslinked polyacrylic acid salt, saponified product of cross-linked acrylic ester-vinyl acetate copolymer, crosslinked product of isobutylene/maleic anhydride copolymer, crosslinked product of polysulfonate salt, and partially cross-linked water-swellable polymers such as polyethylene oxide and polyacrylamide. Among these materials, acrylic acid or acrylate based ones, which are excellent in water absorption amount and water absorption speed, are preferable. In a manufacturing process, water absorbing power and water absorbing speed of the super absorbent resin having the above water-absorbing performance can be adjusted by adjusting the crosslinking density and the crosslinking density gradient.

When the encapsulation sheet 5 that covers the absorber 4 is provided as in this example, the encapsulation sheet 5 is consequently interposed between the liquid-permeable face sheet 3 and the absorber 4. When the encapsulation sheet 5 is made of crepe paper, the encapsulation sheet 5 having excellent absorbency allows rapid diffusion of urine and prevents the urine from returning.

It is preferable that one or both of the absorber 4 and the encapsulation sheet 5 have a color other than white, preferably a relatively dark color having a low lightness such as black or gray. Since the invention relates to an absorbent article for men, the absorbent article gives a calm impression for men by using a relatively dark color and becomes less noticeable when worn in dark-colored underwear, and a trace of urination becomes less noticeable. A coloring method is not particularly limited, and an appropriate dyeing method or a coloring raw material can be used. Further, one or both of the absorber 4 and the encapsulation sheet 5 may be colored by containing activated carbon. As a result, the deodorizing effect of activated carbon can be added.

Three-dimensional deformation guiding grooves 10, 10 ..., which cause three-dimensional deformation of the main body member 6 to conform to the shape of the body when the absorbent article for men 1 is worn, are provided on both sides of the main body member 6 in the vertical direction. The three-dimensional deformation guiding grooves 10, 10 have a substantially line-symmetric shape with respect to the vertical center line CL of the main body member 6, and a plurality of pairs of three-dimensional deformation guiding grooves is provided at intervals in the vertical direction.

As illustrated in Fig. 2, the three-dimensional deformation guiding groove 10 is preferably a groove portion in which the liquid-permeable face sheet 3 and the absorber 4 are integrally recessed from the skin contact surface side toward the non-skin contact surface side, and may be groove portion in which only the absorber 4 or the absorber 4 surrounded by the encapsulation sheet 5 is concavely recessed. Further, the three-dimensional deformation guiding groove 10 may be recessed from the non-skin contact surface side toward the skin contact surface side.

A method based on compression processing can be used to form the three-dimensional deformation guiding groove 10. For example, the three-dimensional deformation guiding groove 10 formed by integrally recessing the liquid-permeable face sheet 3 and the absorber 4 is formed by performing compression processing from the outer surface side of the liquid-permeable face sheet 3 in a state in which the liquid-permeable face sheet 3 is laminated on the skin contact surface side of the absorber 4 in which the groove portion is not formed. In addition, in the case where the three-dimensional deformation guiding groove 10 is formed by concavely recessing only the absorber 4, the three-dimensional deformation guiding groove 10 may be formed by forming a concave groove having a low basis weight of a constituent material of the absorber at a planned formation position of the three-dimensional deformation guiding groove 10.

A pair of right and left three-dimensional deformation guiding grooves 10 is arranged in regions on both sides in the width direction divided in two parts by the vertical center line CL. The three-dimensional deformation guiding grooves 10 and 10 forming a right-left pair are formed in a bilaterally symmetrical shape having a substantially line-symmetrical shape with respect to the vertical center line CL. A plurality of pairs of the right and left three-dimensional deformation guiding grooves 10 and 10 is arranged at intervals in the vertical direction. Specifically, in the example illustrated in Fig. 1, three pairs of 10ₐ, 10_{b}, and 10_{c} are formed in order from the lower side in the vertical direction. Two or more pairs of the three-dimensional deformation guiding grooves 10 may be provided, and two to five pairs are preferable.

In plan view of the absorbent article for men 1 illustrated in Fig. 1, each of the plurality of three-dimensional deformation guiding grooves 10 arranged in the regions on both sides in the width direction is formed in a shape that gradually inclines outward in the width direction toward the upper side in the vertical direction. That is, one three-dimensional deformation guiding groove 10 and the other three-dimensional deformation guiding groove 10 forming a pair are arranged so that a separating distance therebetween in the width direction gradually increases from the lower side to the upper side. In other words, the three-dimensional deformation guiding grooves 10 and 10 forming a pair have a V-shape in plan view from the skin contact surface side in the worn state illustrated in Fig. 1.

The planar shape of each three-dimensional deformation guiding groove 10 is a straight line in the illustrated example. However, each three-dimensional deformation guiding groove 10 may be formed by a curved line, a broken line, a wavy line, etc. bulging upward or downward. Further, in the illustrated example, the three-dimensional deformation guiding groove 10 is formed by a continuous line. However, the three-dimensional deformation guiding groove 10 may be formed by a dotted line or a discontinuous line having a large number of dots arranged at predetermined intervals.

As illustrated in Fig. 1, inclination angles formed between extending directions of the three-dimensional deformation guiding grooves 10 and 10 and a width direction line (αₐ, α_{b}, and α_{c} in order from a three-dimensional deformation guiding groove 10ₐ arranged on the lower side) are formed such that a three-dimensional deformation guiding groove arranged on the lower side has a larger inclination angle than that of a three-dimensional deformation guiding groove arranged on the upper side. That is, the inclination angles have a relationship of αₐ > α_{b} > α_{c}. In plan view illustrated in Fig. 1, while the three-dimensional deformation guiding groove 10ₐ arranged on the lower side has an angle relatively close to an angle of the vertical direction and is arranged in a rather standing state, the three-dimensional deformation guiding groove 10_{c} arranged on the upper side has an angle relatively close to an angle of the width direction and is arranged in a rather lying state.

The inclination angle α_{c} of the three-dimensional deformation guiding groove 10_{c} arranged on the uppermost side is preferably less than 45°, particularly preferably 20° to 40°. In this way, as illustrated in Fig. 3, in a deformed state at the time of wearing, a region on the upper side of the three-dimensional deformation guiding groove 10_{c} is likely to bend to the skin side, and this region is arranged to cover an upper side of a three-dimensional space in which the penis is housed, and is arranged to close the upper side of the three-dimensional space. For this reason, the urine discharged from a tip of the penis is rapidly absorbed by the main body member 6 of the absorbent article for men 1, and leakage can be prevented.

In addition, the inclination angle αₐ of the three-dimensional deformation guiding groove 10ₐ arranged on the lowermost side is preferably 45° or more, particularly preferably 50° to 60°. In this way, a three-dimensional space of a part corresponding to a base end portion of the penis is highly deformed toward the skin side using the three-dimensional deformation guiding groove 10ₐ as a base end, and deformation suitable for the shape of the body is likely to occur.

One three-dimensional deformation guiding groove 10 and the other three-dimensional deformation guiding groove 10 forming a pair are arranged apart to the right and left at a center of the absorbent article for men 1 in the width direction. That is, the three-dimensional deformation guiding groove 10 is arranged in each of the regions on both sides divided in two parts by the vertical center line CL, and does not extend to the region on the opposite side across the vertical center line CL. In this way, in a widthwise central portion of the main body member 6 including the vertical center line CL, a groove-free region in which the three-dimensional deformation guiding groove 10 is not disposed is formed with a predetermined width along the vertical direction.

In a state where the absorbent article for men 1 is worn, as illustrated in Fig. 3, when the penis is applied to the widthwise central portion where the three-dimensional deformation guiding grooves 10 are separated from each other, the main body member 6 is bent from the three-dimensional deformation guiding grooves 10, 10 ... as starting points, the widthwise central portion bulges and is deformed to the non-skin contact surface side, and a three-dimensional space 11 in which the penis is accommodated is formed in the widthwise central portion. Deformation of the widthwise central portion occurs mainly from widthwise inner end edges of the three-dimensional deformation guiding grooves 10, 10 ... or a near position as a starting point. However, since the three-dimensional deformation guiding grooves 10 ... extend obliquely upward toward the outer side in the width direction therefrom, and the three-dimensional deformation guiding groove 10 on the lower side is formed with a larger inclination angle, a bending direction changes stepwise in each part in the vertical direction. That is, on the lower side, a direction in which the three-dimensional deformation guiding groove 10ₐ extends is a direction close to the vertical direction, and thus the main body member 6 is easily bent in the direction. Further, on the upper side, a direction in which the three-dimensional deformation guiding groove 10_{c} extends is a direction close to the width direction, and thus the main body member 6 is easily bent in the direction.

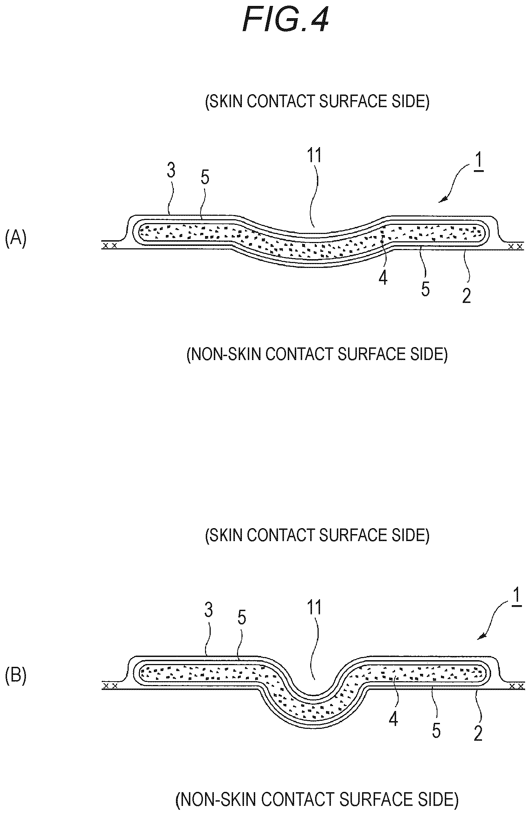

As described above, since the bending direction on both sides of the three-dimensional space 11 changes stepwise with respect to the vertical direction, a cross-sectional shape of the three-dimensional space 11 is an arc having a relatively small radius of curvature toward the non-skin contact surface side and easily bulges high to the non-skin contact surface side with a relatively narrow width as illustrated in Fig. 4(B) on the lower side, and becomes an arc having a gradually larger radius of curvature, is relatively wide, and easily bulges low gradually toward the non-skin contact surface as illustrated in Fig. 4(A) toward the upper side. That is, a planar shape of the three-dimensional space 11 viewed from the skin contact surface side is formed into a fan shape that gradually widens from the lower side to the upper side, and a bulging height to the non-skin side is large on the lower side and gradually decreases toward the upper side. Since the three-dimensional space 11 having such a shape is formed, the penis of the wearer can be accommodated in the three-dimensional space 11 without a feeling of awkwardness, and the main body member 6 can be appropriately fit to the crotch portion of the wearer. Therefore, it is possible to prevent the wearer from feeling awkward or uncomfortable and to prevent leakage of body fluid.

In addition, the body fluid absorbed by the absorber 4 is easily drawn into the three-dimensional deformation guiding groove 10 due to a difference in density of fibers, and is rapidly spread over a wide area along the three-dimensional deformation guiding groove 10, so that the body fluid can be prevented from leaking, and the amount of absorption of the body fluid can be increased.

Furthermore, when the inclination angle α_{c} of the three-dimensional deformation guiding groove 10_{c} located on the uppermost side is less than 45°, as described above, the region on the upper side of the three-dimensional deformation guiding groove 10_{c} is arranged to cover and close the upper side of the three-dimensional space 11, and thus it is possible to reliably prevent leakage from the upper side of the three-dimensional space 11.

From a viewpoint of facilitating the deformation of the three-dimensional space 11, as illustrated in Fig. 1, the three-dimensional deformation guiding groove 10 is preferably formed such that the separating distance in the width direction between a widthwise inner end and the vertical center line CL (Sₐ, S_{b}, and S_{c} in order from the lower side) is smaller for the three-dimensional deformation guiding groove 10 arranged on the lower side than for the three-dimensional deformation guiding groove 10 arranged on the upper side (Sₐ < S_{b} < S_{c}). That is, a minimum separating distance between the three-dimensional deformation guiding grooves 10 and 10 forming a pair is gradually increased toward the upper side. For this reason, the three-dimensional space 11 is easily deformed into a fan shape that gradually widens from the lower side to the upper side in plan view from the skin contact surface side of the absorbent article for men 1.

As illustrated in Fig. 1, a widthwise minimum separating distance T of the three-dimensional deformation guiding grooves 10 and 10 forming the pair is 10 mm or more, preferably 10 to 50 mm. By forming in a size within this range, the penis can be accommodated in the three-dimensional space 11 formed in the widthwise central portion without any awkwardness.

Directions in which a plurality of three-dimensional deformation guiding grooves 10 ... arranged on one side in the width direction among the plurality of pairs of three-dimensional deformation guiding grooves 10, 10 ... extends are arbitrary as long as the relationship of the inclination angles is satisfied. However, as illustrated in Fig. 1, it is particularly preferable to arrange the three-dimensional deformation guiding grooves 10 ... so that extension lines extending from center lines of the three-dimensional deformation guiding grooves 10 outward in the width direction intersect at one point (intersection point A). In this way, both side portions of the three-dimensional space 11 whose widthwise central portion is deformed when the absorbent article for men 1 is worn are easily bent along fold lines radially extending from one predetermined point, and thus the three-dimensional space 11 deformed stepwise is more likely to be formed when compared to the case where the fold lines extend in different directions. A position of the intersection A is arbitrary. However, the intersection A is preferably set at a position outside the main body member 6.

It is preferable that the three-dimensional deformation guiding grooves 10 and 10 adjacent to each other in the vertical direction are arranged at positions not overlapping in the width direction. That is, as illustrated in Fig. 1, between the three-dimensional deformation guiding grooves 10 and 10 adjacent to each other in the vertical direction, a band-shaped region M in which no three-dimensional deformation guiding groove is formed is formed over the entire width in the width direction. When the three-dimensional deformation guiding grooves are formed at positions overlapping in the width direction, a plurality of bent portions is present at the same vertical positions, and there is concern that the widthwise central portion may be hindered from being three-dimensional deformed stepwise with respect to the vertical direction during wearing of the absorbent article for men 1.

In addition, as illustrated in Fig. 1, a length L of the three-dimensional deformation guiding groove 10 in the width direction is preferably 1/4 or more of a length Lₓ in the width direction between an end edge of the three-dimensional deformation guiding groove 10 on the center side in the width direction and a point at which a width direction line extending from an end edge of the three-dimensional deformation guiding groove 10 on the outer side in the width direction intersects with an end edge of the adjacent absorber 4 in the width direction (L ≥ Lₓ/4). In this way, in a vertical section in which the three-dimensional deformation guiding groove 10 is provided, the three-dimensional deformation guiding groove 10 is formed with a predetermined width or more with respect to an absorber part outside the three-dimensional deformation guiding groove 10 in the width direction. Thus, the absorber easily bends from the three-dimensional deformation guiding groove 10 as a starting point, and the three-dimensional space 11 formed in the widthwise central portion is easily formed with a predetermined cross-sectional shape.

It is preferable that the three-dimensional deformation guiding groove 10 is formed in a middle region of the absorber 4, a widthwise outer end of which does not reach an end edge of the absorber 4. In this way, it is possible to prevent the body fluid diffused along the groove from leaking from the end edge of the absorber 4.

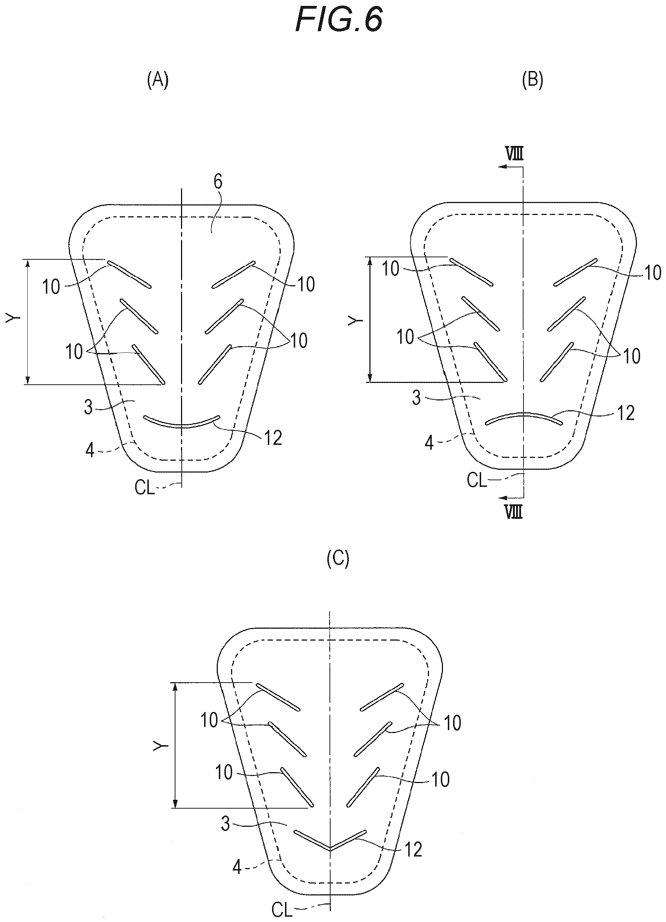

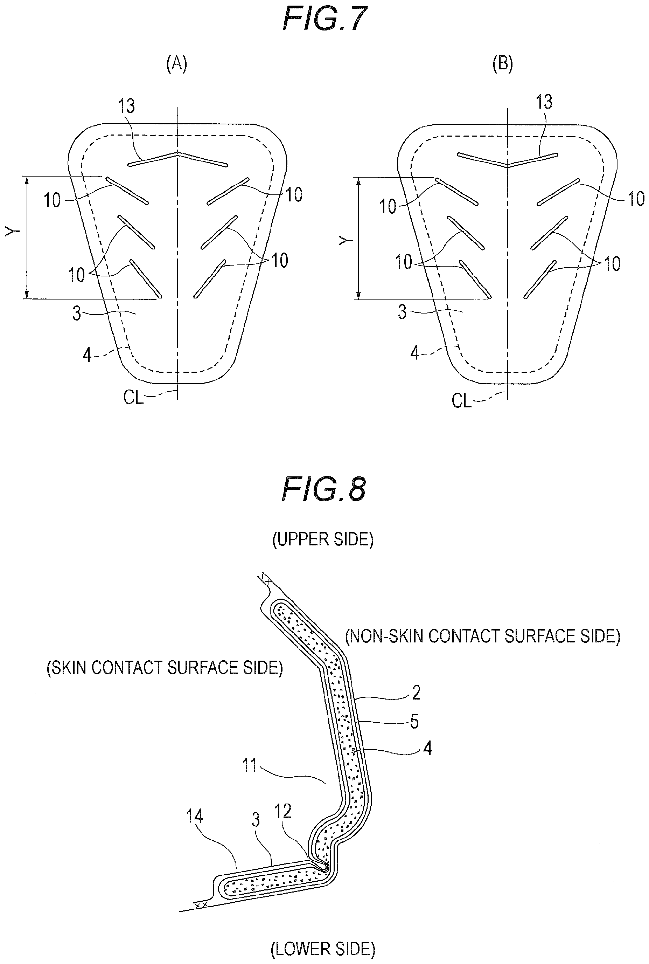

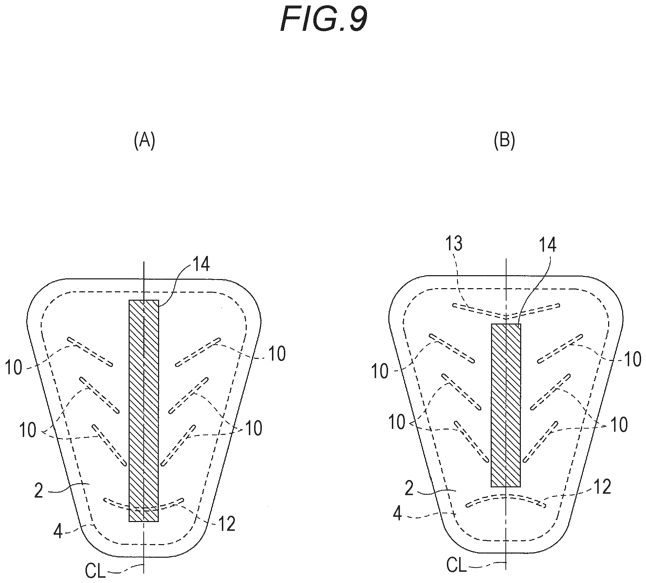

As illustrated in Figs. 6 and 7, in the main body member 6, leakage prevention blocking grooves 12 and 13 extending substantially in the width direction can be arranged in a region on one or both of an upper side and a lower side of a section Y in the vertical direction in which the plurality of pairs of three-dimensional deformation guiding grooves 10, 10 ... is formed. It is preferable that the leakage prevention blocking grooves 12 and 13 are formed to continuously straddle both sides of the vertical center line CL and have a substantially line-symmetric shape with respect to the vertical center line CL. Fig. 6 is an example in which a lower leakage prevention blocking groove 12 is provided in a region on the lower side of the section Y, and Fig. 7 is an example in which an upper leakage prevention blocking groove 13 is provided in a region on the upper side of the section Y Note that even though the groove is provided only in a region on one of the upper side or lower side in the illustrated example, the grooves may be simultaneously provided in regions on both the upper side and the lower side (see Fig. 9(B)).

By providing the leakage prevention blocking grooves 12 and 13, without impairing fitting to the penis, it is possible to block the body fluid flowing upward or downward in the widthwise central portion by the leakage prevention blocking grooves 12 and 13, and to effectively prevent leakage from the end edge of the absorber 4 in the vertical direction.

Planar shapes of the leakage prevention blocking grooves 12 and 13 may be a substantially U-shape or a substantially V-shape curved or bent upward or downward in a convex shape. For example, as illustrated in Figs. 6(A) and 6(C), when the lower leakage prevention blocking groove 12 is formed in a convex shape on the lower side, the body fluid is easily retained in a region of the lower leakage prevention blocking groove 12 protruding downward without impairing fitting of the three-dimensional space 11 to the penis, and diffusion of the body fluid to the lower end edge of the absorber 4 is more reliably suppressed. On the other hand, in the case where the lower leakage prevention blocking groove 12 is formed in a convex shape on the upper side as illustrated in Fig. 6(B), by bending a region on the lower side of the lower leakage prevention blocking groove 12 to the skin side from the lower leakage prevention blocking groove 12 as a starting point as illustrated in Fig. 8 in a state in which the three-dimensional space 11 is formed in the widthwise central portion during wearing of the absorbent article for men 1, a scrotum support portion 14 that supports the scrotum is formed on the lower side of the three-dimensional space 11. Thus, wearing feeling becomes more excellent, and leakage from the lower side can be more reliably prevented.

In addition, as illustrated in Fig. 7(A), when a planar shape of the upper leakage prevention blocking groove 13 is formed in an upwardly convex shape in plan view, the body fluid is more likely to be retained in a region of the upper leakage prevention blocking groove 13 protruding upward, and diffusion of the body fluid to the upper end edge of the absorber 4 can be more reliably suppressed. Meanwhile, as illustrated in Fig. 7(B), when the upper leakage prevention blocking groove 13 is formed in a downwardly convex shape, a region on the upper side of the upper leakage prevention blocking groove 13 is easily bent toward the skin side from the upper leakage prevention blocking groove 13 as a starting point.

A shift prevention layer 14 for fixing to the underwear is preferably provided on the non-skin contact surface of the absorbent article for men 1. A known form can be appropriately used as the shift prevention layer 14. However, an adhesive is preferably used. When the shift prevention layer 14 contains the adhesive, the shift prevention layer 14 is covered with a release sheet (not illustrated) so that peeling is allowed in a state before the absorbent article for men 1 is used.

The shift prevention layer 14 can be arranged in any form, and is preferably provided along the vertical direction in a part overlapping the widthwise central portion, from which the three-dimensional deformation guiding grooves 10 and 10 forming the pair are separated to the right and left, in a thickness direction as illustrated in Fig. 9. In this way, it is possible to prevent deformation of the main body member 6 from being hindered by the shift prevention layer 14 when the absorbent article for men 1 is worn.

As illustrated in Fig. 9(A), the shift prevention layer 14 may be formed at a position overlapping the leakage prevention blocking grooves 12 and 13 formed in the upper and lower parts in the thickness direction as long as the shift prevention layer 14 is formed in the widthwise central portion not overlapping the three-dimensional deformation guiding grooves 10, 10 ... in the thickness direction. However, when the leakage prevention blocking grooves 12 and 13 are used as starting points of deformation of the main body member 6, the shift prevention layer 14 is preferably formed in a region not overlapping the leakage prevention blocking grooves 12 and 13 in the thickness direction so as not to hinder deformation of the main body member 6 from the leakage prevention blocking grooves 12 and 13 as the starting points as illustrated in Fig. 9(B). The shift prevention layer 14 may be formed by one strip that is continuous in the vertical direction as in the illustrated example, formed by a plurality of strips separated in the width direction, or formed by a discontinuous line in the vertical direction separated in the vertical direction at a position overlapping the leakage prevention blocking grooves 12 and 13, etc.

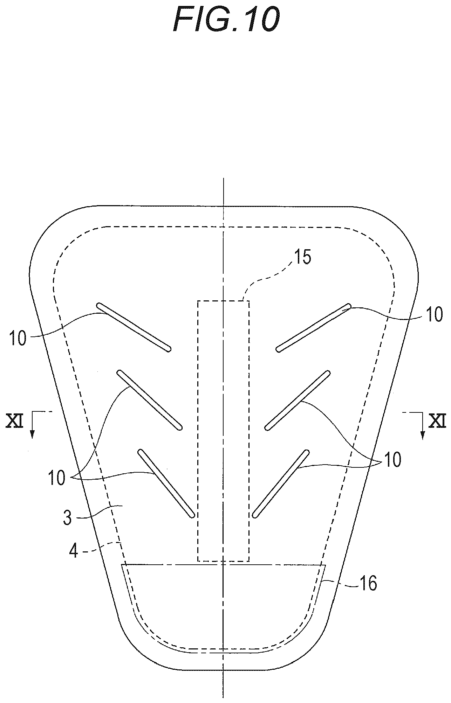

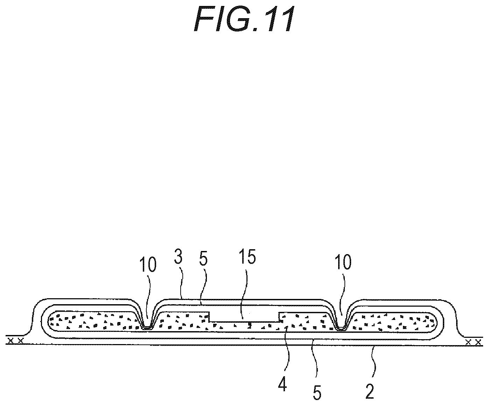

In the absorber 4, as illustrated in Figs. 10 and 11, a low basis weight portion 15 in which a basis weight of a constituent material of the absorber 4 (pulp and super absorbent resin) is relatively low may be formed along the vertical direction in the widthwise central portion from which the three-dimensional deformation guiding grooves 10 and 10 forming the pair are separated to the right and left. The low basis weight portion 15 has a lower basis weight than that of other absorber portions, and thus has low rigidity and is easily deformed. Therefore, when the absorbent article for men 1 is worn, the low basis weight portion 15 is likely to be three-dimensionally deformed, and the penis is easily fit to a three-dimensionally deformed portion.

The basis weight of the low basis weight portion 15 is 50% or less, preferably 20 to 50% of the basis weight of the other absorber portion. By having such a difference in basis weight, the above effect can be more reliably exhibited.

When the low basis weight portion 15 is provided, as illustrated in Fig. 10, a region 16 on the lower side of the low basis weight portion 15 preferably has a relatively higher basis weight of the constituent material of the absorber 4 than that of the absorber portion other than the low basis weight portion 15. In this way, since the amount of body fluid that can be absorbed in the region 16 on the lower side increases, it is possible to prevent the body fluid from leaking from the region 16 on the lower side since the ability to absorb the body fluid decreases in the low basis weight portion 15 and the body fluid easily flows to a region on the lower side of the low basis weight portion 15.

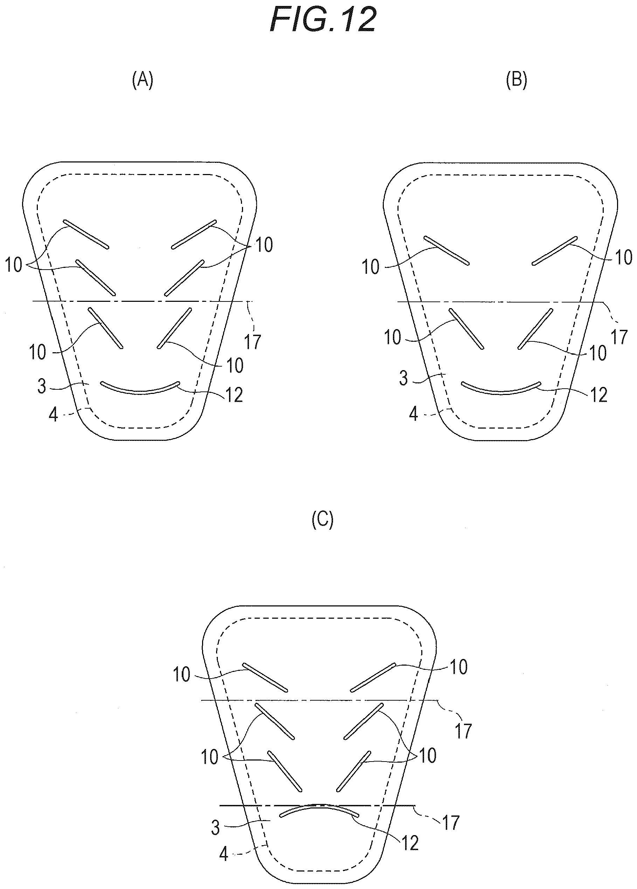

As illustrated in Fig. 12, in an item packaging state, the absorbent article for men 1 is preferably folded in the vertical direction at one or a plurality of fold lines 17 spaced in the vertical direction along the width direction. In this instance, the fold line 17 is preferably provided at a position not intersecting the three-dimensional deformation guiding groove 10. That is, the fold line 17 is arranged between the vertically adjacent three-dimensional deformation guiding grooves 10 and 10 or above or below a longitudinal section in which a plurality of pairs of three-dimensional deformation guiding grooves 10 is formed. As illustrated in Figs. 12(A) and 12(B), when one fold line 17 is provided at a central portion in the vertical direction, the fold line 17 is preferably arranged between three-dimensional deformation guiding grooves 10 and 10 adjacent to each other in the vertical direction. In addition, as illustrated in Fig. 12(C), a plurality of (two in the illustrated example) fold lines 17 and 17 is provided at an interval in the vertical direction, the fold lines 17 are preferably arranged between three-dimensional deformation guiding grooves 10 and 10 adjacent to each other in the vertical direction and below a longitudinal section in which the plurality of pairs of three-dimensional deformation guiding grooves 10 ... is formed, respectively. Here, when the lower leakage prevention blocking groove 12 is provided as in the illustrated example, the fold line 17 arranged below the longitudinal section in which the plurality of three-dimensional deformation guiding grooves 10 ... is formed may be arranged at a position overlapping the lower leakage prevention blocking groove 12. In particular, by making a top position where the lower leakage prevention blocking groove 12 bulges in the vertical direction substantially coincide with the fold line 17, the region below the lower leakage prevention blocking groove 12 is easily bent to the skin side during wearing by a crease at the time of folding at the fold line 17, which is preferable.

### Reference Signs List

- 1: Absorbent article for men
- 2: Liquid-impermeable back sheet
- 3: Liquid-permeable face sheet
- 4: Absorber
- 5: Encapsulation sheet
- 6: Main body member
- 10: Three-dimensional deformation guiding groove
- 11: Three-dimensional space
- 12: Lower leakage prevention blocking groove
- 13: Upper leakage prevention blocking groove
- 14: Shift prevention layer
- 15: Low basis weight portion
- 17: Fold line

## Claims

1. An absorbent article (1) for men having a vertical direction and a width direction orthogonal to the vertical direction, the absorbent article comprising
a main body member (6) including an absorber,
**characterized in that**:
the main body member comprises a plurality of pairs of three-dimensional deformation guiding grooves (10) having a substantially line-symmetric shape with respect to a vertical center line (CL) of the main body member, the plurality of pairs of three-dimensional deformation guiding grooves being provided at intervals in the vertical direction in regions on both sides in the width direction divided in two parts by the vertical center line (CL);
each of the three-dimensional deformation guiding grooves is formed in a shape gradually inclined outward in the width direction toward an upper side, and an inclination angle of a three-dimensional deformation guiding groove arranged on a lower side is larger than an inclination angle of a three-dimensional deformation guiding groove arranged on the upper side, the inclination angle being an angle formed by a direction in which the three-dimensional deformation guiding groove extends and a width direction line; and
one three-dimensional deformation guiding groove and the other three-dimensional deformation guiding groove forming a pair are arranged apart to a right and a left at a center of the absorbent article for men in the width direction.

2. The absorbent article for men according to claim 1, wherein a separating distance of a three-dimensional deformation guiding groove arranged on the lower side is smaller than a separating distance of a three-dimensional deformation guiding groove arranged on the upper side, the separating distance being a distance in the width direction between a widthwise inner end of the three-dimensional deformation guiding groove and the vertical center line.

3. The absorbent article for men according to claim 1 or 2, wherein three-dimensional deformation guiding grooves adjacent to each other in the vertical direction are arranged at positions not overlapping in the width direction.

4. The absorbent article for men according to any one of claims 1 to 3, wherein a leakage prevention blocking groove (12, 13) extending substantially in the width direction is arranged in the main body member in one or both of regions on an upper side or a lower side of a vertical section in which the plurality of pairs of three-dimensional deformation guiding grooves is formed.

5. The absorbent article for men according to any one of claims 1 to 4,
wherein a shift prevention layer for fixing to underwear is provided on a non-skin contact surface of the absorbent article for men, and
the shift prevention layer (14) is provided along the vertical direction in a part overlapping a widthwise central portion, from which the three-dimensional deformation guiding grooves forming the pair are separated to the right and left, in a thickness direction.

6. The absorbent article for men according to any one of claims 1 to 5, wherein in the absorber, a low basis weight portion (15) is formed along the vertical direction in the widthwise central portion, from which the three-dimensional deformation guiding grooves forming the pair are separated to the right and left.

7. The absorbent article for men according to any one of claims 1 to 6,
wherein the absorbent article for men is folded in the vertical direction at one or a plurality of fold lines (17) spaced in the vertical direction along the width direction in an item packaging state, and
the fold line (17) is provided at a position not intersecting the three-dimensional deformation guiding groove.

## Patentansprüche

1. Saugfähiger Artikel (1) für Männer, aufweisend eine vertikale Richtung und eine Breitenrichtung orthogonal zur vertikalen Richtung, wobei der saugfähige Artikel Folgendes umfasst:
ein Hauptkörperelement (6), darin eingeschlossen einen Absorber,
**dadurch gekennzeichnet, dass**:
das Hauptkörperelement eine Vielzahl von Paaren von dreidimensionalen Verformungsführungsrillen (10) mit einer im Wesentlichen liniensymmetrischen Form mit Bezug auf die vertikalen Mittellinie (CL) des Hauptkörperelements umfasst, wobei die Vielzahl von Paaren von dreidimensionalen Verformungsführungsrillen in Intervallen in der vertikalen Richtung in Regionen auf beiden Seiten in der Breitenrichtung, unterteilt in zwei Teile durch die vertikale Mittellinie (CL), bereitgestellt ist;
jede der dreidimensionalen Verformungsführungsrillen in einer Form gebildet ist, die allmählich nach außen in der Breitenrichtung hin zu einer oberen Seite geneigt ist, und ein Neigungswinkel einer dreidimensionalen Verformungsführungsrille, angeordnet auf einer unteren Seite, größer als ein Neigungswinkel einer dreidimensionalen Verformungsführungsrille ist, die auf der oberen Seite angeordnet ist, wobei der Neigungswinkel ein Winkel ist, der von einer Richtung, in die sich die dreidimensionale Verformungsführungsrille erstreckt, und einer Breitenrichtungslinie gebildet ist; und
eine dreidimensionale Verformungsführungsrille und die andere dreidimensionale Verformungsführungsrille, die ein Paar bilden, voneinander beabstandet rechts und links an einer Mitte des saugfähigen Artikels für Männer in der Breitenrichtung angeordnet sind.

2. Saugfähiger Artikel für Männer nach Anspruch 1 wobei eine trennende Distanz einer dreidimensionalen Verformungsführungsrille, die auf der unteren Seite angeordnet ist, kleiner als eine trennende Distanz einer dreidimensionalen Verformungsführungsrille ist, die auf der oberen Seite angeordnet ist, wobei die trennende Distanz eine Distanz in der Breitenrichtung zwischen einem der Breite nach inneren Ende der dreidimensionalen Verformungsführungsrille und der vertikalen Mittellinie ist.

3. Saugfähiger Artikel für Männer nach Anspruch 1 oder 2, wobei dreidimensionale Verformungsführungsrillen benachbart zueinander in der vertikalen Richtung an Positionen angeordnet sind, die in der Breitenrichtung nicht überlappen.

4. Saugfähiger Artikel für Männer nach einem der Ansprüche 1 bis 3, wobei eine Auslaufpräventions-Blockierrille (12, 13), die sich im Wesentlichen in der Breitenrichtung erstreckt, im Hauptkörperelement in einer oder in beiden Regionen auf einer oberen Seite oder einer unteren Seite einer vertikalen Sektion angeordnet ist, in der die Vielzahl von Paaren von dreidimensionalen Verformungsführungsrillen gebildet ist.

5. Saugfähiger Artikel für Männer nach einem der Ansprüche 1 bis 4, wobei eine Rutschpräventionsschicht zur Befestigung an der Unterwäsche auf einer Nicht-Haut-Kontaktfläche des saugfähigen Artikels für Männer bereitgestellt ist, und die Rutschpräventionsschicht (14) entlang der vertikalen Richtung in einem Teil bereitgestellt ist, der einen der Breite nach zentralen Abschnitt überlappt, von dem die dreidimensionalen Verformungsführungsrillen, die das Paar bilden, links und rechts in einer Dickenrichtung getrennt sind.

6. Saugfähiger Artikel für Männer nach einem der Ansprüche 1 bis 5, wobei im Absorber ein Gewichtsabschnitt (15) der unteren Basis entlang der vertikalen Richtung im zentralen Abschnitt der Breite nach gebildet ist, von dem die dreidimensionalen Verformungsführungsrillen, die das Paar bilden, rechts und links getrennt sind.

7. Saugfähiger Artikel für Männer nach einem der Ansprüche 1 bis 6, wobei der saugfähige Artikel für Männer in der vertikalen Richtung an einer oder einer Vielzahl von Faltlinie(n) (17) gefaltet ist, die in der vertikalen Richtung entlang der Breitenrichtung in einem Packstückzustand beabstandet ist, und die Faltlinie (17) an einer Position bereitgestellt ist, die die dreidimensionale Verformungsführungsrille nicht schneidet.

## Revendications

1. Article absorbant (1) pour hommes ayant une direction verticale et une direction de largeur orthogonale à la direction verticale, l'article absorbant comprenant :
un élément de corps principal (6) comprenant un absorbeur,
**caractérisé en ce que** :
l'élément de corps principal comprend une pluralité de paires de rainures de guidage de déformation tridimensionnelles (10) ayant une forme de symétrie sensiblement linéaire par rapport à une ligne centrale verticale (CL) de l'élément de corps principal, la pluralité de paires de rainures de guidage de déformation tridimensionnelles étant prévues à intervalles dans la direction verticale dans des régions des deux côtés dans la direction de la largeur, divisées en deux parties par la ligne centrale verticale (CL) ;
chacune des rainures de guidage de déformation tridimensionnelles est formée en une forme progressivement inclinée vers l'extérieur dans la direction de la largeur vers un côté supérieur, et un angle d'inclinaison d'une rainure de guidage de déformation tridimensionnelle agencée sur un côté inférieur est supérieur à un angle d'inclinaison d'une rainure de guidage de déformation tridimensionnelle sur le côté supérieur, l'angle d'inclinaison étant un angle formé par une direction dans laquelle la rainure de guidage de déformation tridimensionnelle s'étend et une ligne dans la direction de la largeur ; et
une rainure de guidage de déformation tridimensionnelle et l'autre rainure de guidage de déformation tridimensionnelle formant une paire sont agencées à droite et à gauche au niveau d'un centre de l'article absorbant pour hommes dans la direction de la largeur.

2. Article absorbant pour hommes selon la revendication 1, dans lequel une distance de séparation d'une rainure de guidage de déformation tridimensionnelle agencée sur un côté inférieur est inférieure à une distance de séparation d'une rainure de guidage de déformation tridimensionnelle agencée sur le côté supérieur, la distance de séparation étant une distance dans la direction de la largeur entre une extrémité interne dans le sens de la largeur de la rainure de guidage de déformation tridimensionnelle et la ligne centrale verticale.

3. Article absorbant pour hommes selon la revendication 1 ou 2, dans lequel les rainures de guidage de déformation tridimensionnelles adjacentes entre elles dans la direction verticale sont agencées dans des positions qui ne se chevauchent pas la direction de la largeur.

4. Article absorbant pour hommes selon l'une quelconque des revendications 1 à 3, dans lequel une rainure de blocage antifuite (12, 13) s'étendant sensiblement dans la direction de la largeur est agencée dans l'élément de corps principal dans une ou deux des régions sur un côté supérieur ou un côté inférieur d'une section verticale dans laquelle est formée la pluralité de paires de rainures de guidage de déformation tridimensionnelles.

5. Article absorbant pour hommes selon l'une quelconque des revendications 1 à 4,
dans lequel une couche antidéplacement pour la fixation sur le sous-vêtement est prévue sur une surface sans contact avec la peau de l'article absorbant pour hommes, et
la couche antidéplacement (14) est prévue le long de la direction verticale dans une partie chevauchant sur une partie centrale dans le sens de la largeur, à partir de laquelle les rainures de guidage de déformation tridimensionnelles formant la paire sont séparées à droite et à gauche, dans une direction d'épaisseur.

6. Article absorbant pour hommes selon l'une quelconque des revendications 1 à 5, dans lequel, dans l'absorbeur, une partie de faible poids de base (15) est formée le long de la direction verticale dans la partie centrale dans le sens de la largeur, à partir de laquelle les rainures de guidage de déformation tridimensionnelles formant la paire sont séparées à droite et à gauche.

7. Article absorbant pour hommes selon l'une quelconque des revendications 1 à 6,
dans lequel l'article absorbant pour hommes est plié dans la direction verticale au niveau d'une ou d'une pluralité de lignes de pliage (17) espacées dans la direction verticale le long de la direction de la largeur dans un état d'emballage d'article, et
la ligne de pliage (17) est prévue dans une position ne coupant pas la rainure de guidage de déformation tridimensionnelle.
